# EUROPEAN PATENT APPLICATION

(11) **EP 4 796 140 A1**
(43) Date of publication of application: **26.08.2026**
(21) Application number: 24878596.6
(22) Date of filing: 24.07.2024
(51) Int. Cl.: A61M 1/28

(54) **PERITONEAL DIALYSIS APPARATUS**

(30) Priority: 18.10.2023 CN 202322800211 U
(71) Applicant: VR Medical Technology Co., Ltd., Suzhou, Jiangsu 215325 (CN)
(72) Inventor: PAN, Li, Suzhou, Jiangsu 215325 (CN); LI, Zhou, Suzhou, Jiangsu 215325 (CN); HUANG, Xiaodong, Suzhou, Jiangsu 215325 (CN)
(74) Representative: Herrero & Asociados, S.L.
(86) International application number: PCT/CN2024/107247
(87) International publication number: WO 2025/081944

(57) **Abstract**

The present invention relates to the technical field of peritoneal dialysis, and in particular to a peritoneal dialysis apparatus. Disclosed in the present invention is a peritoneal dialysis apparatus for being used in combination with a liquid transfer cavity for peritoneal dialysis. The peritoneal dialysis apparatus comprises a driving unit, the liquid transfer cavity comprises a pressure cavity, the pressure cavity is in fluid communication with the driving unit, and by changing the pressure in the pressure cavity, the driving unit controls liquid to enter and exit the pressure cavity through a liquid pipeline. The peritoneal dialysis apparatus of the present invention has a simple control principle, a reliable structure, a faster and more stable flow guide speed, and a good treatment effect, produces less noise, and can improve the treatment experience of patients.

## Description

This application claims the benefit of priority to Chinese Patent Application No. 202322800211.0, filed on Oct. 18, 2023, and entitled "Peritoneal Dialysis Apparatus and Control Method thereof," the entire contents of which are incorporated herein by reference.

### FIELD OF TECHNOLOGY

The present invention relates to the technical field of peritoneal dialysis, and in particular to a peritoneal dialysis apparatus.

### BACKGROUND

Peritoneal dialysis (abbreviated as "PD") achieves, through exchange between a peritoneal dialysis medical solution and human body fluids, the removal of metabolic waste products and toxic substances as well as the correction of water and electrolyte imbalances, by utilizing a peritoneum within a patient's abdominal cavity as a semipermeable membrane and instilling a peritoneal dialysis medical solution into the peritoneal cavity via a catheter, based on the principle that a concentration gradient difference across the peritoneum drives solutes from a higher-concentration side to a lower-concentration side (diffusion) and water from a hypotonic side to a hypertonic side (osmosis). Common peritoneal dialysis modalities include continuous ambulatory peritoneal dialysis (CAPD), intermittent peritoneal dialysis (IPD), continuous cyclic peritoneal dialysis (CCPD), nocturnal intermittent peritoneal dialysis (NIPD), and tidal peritoneal dialysis (TPD).

Traditional peritoneal dialysis treatment relies on manual operation, which involves multiple steps and is time-consuming and labor-consuming. Automatic peritoneal dialysis (APD) automates a peritoneal dialysis process through an automated cyclic peritoneal dialysis apparatus controlled by computer programs, and can flexibly adopt different dialysis modes according to patient needs and clinical characteristics. APD apparatuses are convenient to use, provide patients with more opportunities for free movement, and are increasingly accepted and adopted by a growing number of patients.

In the prior art, common automatic peritoneal dialysis apparatuses can be classified into gravity-based peritoneal dialysis apparatuses (referred to as gravity-based APD) and powered peritoneal dialysis apparatuses (referred to as powered APD). The gravity-based APD apparatuses mainly utilize gravity to drive liquid to flow, without using other mechanical power or with only mechanical power as auxiliary power. The powered APD apparatuses mainly utilize the mechanical power to drive liquid to flow, causing a dialysate to flow within a dialysis pipeline and form a dialysate stream.

Existing powered APD apparatuses mainly operate on a membrane-cavity pressure-driven principle, wherein a membrane liquid cavity and a labyrinth liquid passage are provided in a disposable cassette. The membrane liquid cavity has a flexible membrane as a side wall of the cavity. The apparatus uses a gas control component such as an air pump and an air tank to deform the flexible membrane, transmits pressure through the flexible membrane, and alternately generates positive or negative pressure within the membrane liquid cavity to drive the dialysate to flow in a dialysis pipeline, and controls the opening and closing of the liquid channel by adjusting air pressure to cause the membrane to occlude the liquid channel. The air pump needs to operate for a long time to continuously drive the liquid flow, and since the driving force for the liquid flow comes from the periodic pressure changes in the membrane liquid cavity, the traditional powered APD apparatuses experience frequent pressure fluctuations in the pipeline during the dialysate flow, resulting in unstable and slow flow rates, high failure rates, and significant noise levels.

Current gravity-based APD mainly relies on gravitational height differences to achieve fluid flow. Some apparatuses are supplemented with a peristaltic pump to provide added power. The liquid flow rate is affected by factors such as a height of a dialysis bag, leading to an unstable liquid flow rate and a slower drainage process. Therefore, for the same treatment prescription, the gravity-based APD apparatuses typically requires a longer treatment time than the powered APD apparatuses.

### SUMMARY

An objective of the present invention is to provide a peritoneal dialysis apparatus to address shortcomings or defects in the prior art.

The peritoneal dialysis apparatus of the present invention eliminates the disposable cassette with a membrane and related control mechanisms. A liquid transfer cavity is connected to a driving unit through a connection interface, so that the driving unit is utilized to directly change the pressure within the liquid transfer cavity, thereby controlling the flow of liquid within the drainage pipeline. The peritoneal dialysis apparatus of the present invention has a simple control principle, a reliable structure, a faster and more stable drainage speed, a good treatment effect, produces a low noise level, and can improve the treatment experience of the patient.

In a first aspect, the present invention discloses a peritoneal dialysis apparatus for being used in combination with a liquid transfer cavity for peritoneal dialysis, the peritoneal dialysis apparatus including a driving unit, wherein
the liquid transfer cavity includes a pressure cavity, the pressure cavity is in fluid communication with the driving unit, and by changing a pressure in the pressure cavity, the driving unit controls liquid to enter and exit the pressure cavity through a liquid pipeline.

The liquid transfer cavity can be connected to a heating bag, a waste fluid collector, and a human peritoneal cavity through the liquid pipeline respectively. Specifically, the liquid pipeline may include a medical solution bag pipeline and/or a drainage pipeline. For example, the medical solution bag pipeline may include a liquid supply tube, a liquid supplementation tube, or a last tube, and the drainage pipeline may include a human body tube and a waste fluid tube. Optionally, the liquid pipeline may further include branch tubes for auxiliary connection.

The liquid supply tube, the liquid supplementation tube, and the last tube are configured to connect the heating bag, the liquid supplementation bag, and the last bag, respectively. The human body tube is configured to be communicated with the human peritoneal cavity. Specifically, a peritoneal dialysis indwelling catheter is implanted in the abdominal cavity of a subject undergoing peritoneal dialysis, an extracorporeal portion of the peritoneal dialysis indwelling catheter is connected to a peritoneal dialysis extension set, and the human body tube of the liquid transfer cavity is used for connecting with the peritoneal dialysis extension set so as to communicate with the human peritoneal cavity. The waste fluid tube is configured to be connected to the waste fluid collector.

In some implementations, the driving unit changes the pressure in the pressure cavity by injecting gas into or withdrawing gas from the pressure cavity. Specifically, the pressure cavity has a connection interface to be in fluid communication with the driving unit, and gas can enter or exit the pressure cavity through the connection interface. Ideally, the gas is air, including filtered air.

In some implementations, the liquid includes either or both of a peritoneal dialysis medical solution and a body waste fluid. Specifically, in a drainage process, the liquid is a body waste fluid; and in a powered infusion process or powered liquid supplementation process, the liquid is clean peritoneal dialysis medical solution.

In some implementations, the driving unit includes a pressure control mechanism, the pressure control mechanism includes a pressure generator and an air pressure detector, the pressure generator is configured to change the pressure in the pressure cavity, and the air pressure detector is configured to monitor the pressure in the pressure cavity. By means of the pressure generator and the air pressure detector, the driving unit can adjust the pressure in the pressure cavity to a specific value or range as needed, providing a stable and reliable power source for the inflow or outflow of the medical solution or the waste fluid from the pressure cavity. In particular, the pressure generator may be an air pump, for example, a membrane pump.

In some implementations, the peritoneal dialysis apparatus further includes a pipeline switching valve assembly, and the pipeline switching valve assembly is configured to control communication or closure of the liquid pipeline. Specifically, the pipeline switching valve assembly may include a plurality of pipeline switching valves, and the pipeline switching valves can independently control the communication or closure of the respective liquid pipelines. For example, the pipeline switching valve assembly may control communication or closure of any one or more of the medical solution bag pipeline, the drainage pipeline, and the branch tubes.

Preferably, the pipeline switching valve may include a solenoid valve or a motor-driven valve. More preferably, the pipeline switching valve may include a motor-driven valve.

In some implementations, the driving unit operates at least in a drainage process to discharge the body waste fluid out of a human body.

In some implementations, the operation of the driving unit is not essential in a medical solution infusion process. In other words, in the medical solution infusion process, the driving unit operates optionally. Depending on a specific infusion mode of the medical solution infusion process, the driving unit may or may not operate. Specifically, the driving unit does not operate in a gravity-based infusion mode; and the driving unit operates in a powered infusion mode.

In some implementations, the peritoneal dialysis apparatus further includes a first weight detector for measuring a weight of a heating bag. Optionally, the peritoneal dialysis apparatus further includes a heating tray for supporting and heating the heating bag.

In some implementations, the peritoneal dialysis apparatus further includes a second weight detector for measuring a weight of liquid in the pressure cavity. Preferably, the second weight detector may measure the weight of the liquid in the pressure cavity indirectly, for example, by calculating a weight difference.

In some implementations, the peritoneal dialysis apparatus further includes a housing, and a compartment for mounting the liquid transfer cavity is disposed within the housing. In a peritoneal dialysis process, the pressure cavity of the liquid transfer cavity is not accessible from outside the apparatus.

Preferably, the first weight detector or the second weight detector may be provided within the housing.

In some implementations, the liquid pipeline includes a medical solution bag pipeline, a human body tube, and branch tubes, the medical solution bag pipeline and the human body tube are connected to the pressure cavity through a same branch tube, and the medical solution bag pipeline is capable of communicating with the human body tube without passing through the pressure cavity. Thus, the peritoneal dialysis apparatus can utilize gravity for medical solution infusion.

Preferably, the medical solution bag pipelines can communicate with each other without passing through the pressure cavity, whereby liquid supplementation from the liquid supplementation bag or the last bag to the heating bag can be accomplished by gravity.

In some implementations, the liquid transfer cavity further includes a connection cavity, the connection cavity is not directly communicated with the pressure cavity, and the medical solution bag pipeline is communicated with the human body tube through the connection cavity. Specifically, the connection cavity is in fluid communication with one or more of the heating bag, the liquid supplementation bag, and the last bag through the medical solution bag pipeline respectively.

As used herein, the connection cavity and the pressure cavity are "not directly communicated," meaning that a channel between the connection cavity and the pressure cavity can be switched to a closed state. For example, the connection cavity and the pressure cavity may be communicated through one or more branch tubes, and these branch tubes can be switched on or off by pipeline switches. As another example, the connection cavity and the pressure cavity are connected through a switchable valve.

In some implementations, the liquid pipeline further includes a waste fluid tube, and the waste fluid tube is directly connected to the pressure cavity, or the waste fluid tube is connected to the pressure cavity through the branch tube. Preferably, the waste fluid tube is directly connected to the pressure cavity.

In some implementations, one end, close to the liquid transfer cavity, of the human body tube is connected to the connection cavity or the medical solution bag pipeline through a first branch tube, and connected to the pressure cavity through a second branch tube, and the other end, away from the liquid transfer cavity, of the human body tube is configured to be connected to the human peritoneal cavity, one end of the waste fluid tube is connected to the pressure cavity, and the other end of the waste fluid tube is configured to be connected to the waste fluid collector.

In some implementations, a volume of the pressure cavity is independent of the pressure in the pressure cavity. Specifically, a volume of the pressure cavity remains substantially unchanged within a working pressure range of the pressure cavity; for example, a change rate of the volume of the pressure cavity may be less than 5%, less than 2%, or less than 1%. Preferably, a cavity body portion of the liquid transfer cavity may be made of a rigid material, and a side wall of the pressure cavity does not undergo significant deformation within the working pressure range.

In some implementations, the volume of the pressure cavity is 20-500 mL, for example, 20 mL, 30 mL, 40 mL, 50 mL, 80 mL, 100 mL, 150 mL, 200 mL, 300 mL, 500 mL, or any range between any two of these values, preferably 30-300 mL, more preferably 50-200 mL. A reasonable volume facilitates stable and rapid control of an internal pressure of the pressure cavity. The volume may be a maximum volume of gas or liquid that the pressure cavity can actually accommodate, or a maximum volume of gas or liquid that the pressure cavity is designed to accommodate.

In some implementations, the peritoneal dialysis apparatus may include a gas-permeable and water-blocking element to prevent liquid from entering the driving unit from the pressure cavity. Preferably, the gas-permeable and water-blocking and water-blocking element is disposed at a position where the pressure cavity is connected to the driving unit. Specifically, the gas-permeable and water-blocking membrane may be disposed at the connection interface to prevent liquid from passing through the connection interface.

In some implementations, the peritoneal dialysis apparatus may include a bacteria-blocking element to prevent bacteria and other pathogens in the air from entering the pressure cavity through the connection interface. Preferably, the bacteria-blocking element is disposed at the connection interface.

Preferably, the gas-permeable and water-blocking and water-blocking element and the bacteria-blocking element are the same, for example, a dual-function element that is gas-permeable and water-blocking, water-blocking, and bacteria-blocking. More specifically, the dual-function element is a water-blocking and bacteria-blocking membrane.

In some implementations, the peritoneal dialysis apparatus further includes a data processing unit, and the data processing unit is configured to receive and process data from the first weight detector and/or the second weight detector.

In some implementations, the data processing unit may be in communication link with the pressure control mechanism. Specifically, the data processing unit may receive pressure data from the pressure control mechanism or send pressure control signals to the pressure control mechanism.

In a second aspect, the present invention discloses a peritoneal dialysis apparatus for being used in combination with a liquid transfer cavity for peritoneal dialysis, wherein the liquid transfer cavity is connectable to a waste fluid collector and a human peritoneal cavity through a liquid pipeline, and the peritoneal dialysis apparatus includes a second weight detector for measuring a weight of liquid within the liquid transfer cavity.

In some implementations, the liquid transfer cavity includes a pressure cavity, the pressure cavity is connected to the liquid pipeline, and the pressure within the pressure cavity can be monitored and regulated to control liquid to enter and exit the pressure cavity through the liquid pipeline. Specifically, the pressure cavity may be directly or indirectly connected to the liquid pipeline.

In some implementations, the peritoneal dialysis apparatus includes a pressure control mechanism, and the pressure control mechanism is configured to monitor and control the pressure within the pressure cavity. Specifically, the pressure control mechanism includes a pressure generator and an air pressure detector, the pressure generator is configured to change the pressure within the pressure cavity, and the air pressure detector is configured to monitor the pressure within the pressure cavity.

In some embodiments, the peritoneal dialysis apparatus further includes a pipeline switching valve assembly, and the pipeline switching valve assembly is configured to control communication or closure of the liquid pipeline.

In some implementations, the peritoneal dialysis apparatus further includes a housing, and a compartment for mounting the liquid transfer cavity is disposed within the housing.

In some implementations, the liquid pipeline may include a medical solution bag pipeline and/or a drainage pipeline, the medical solution bag pipeline may include a liquid supply tube, an optional liquid supplementation tube, and an optional last tube, and the drainage pipeline may include a human body tube and a waste fluid tube. Optionally, the liquid pipeline may further include branch tubes.

In some implementations, the medical solution bag pipeline may be communicated with the human body tube without passing through the pressure cavity.

In a third aspect, the present invention discloses a peritoneal dialysis system, including:
a liquid transfer cavity having a sealed pressure cavity;
a human body tube communicating the pressure cavity with a human peritoneal cavity; and
a driving unit for providing mechanical power, the driving unit being in fluid communication with the pressure cavity;
wherein the peritoneal dialysis system is capable of utilizing the mechanical power to discharge the body waste fluid from the human peritoneal cavity out of the human body.

In some implementations, the peritoneal dialysis system further includes a medical solution bag pipeline that communicates the solution bag with the human body tube. Specifically, the medical solution bag includes at least a heating bag, and the medical solution bag pipeline includes at least a liquid supply tube.

In some implementations, the peritoneal dialysis system is capable of using gravity or mechanical power to infuse the medical solution into the human peritoneal cavity through the human body tube.

In some implementations, the driving unit applies the mechanical power by injecting gas into or withdrawing gas from the liquid transfer cavity. Further, the driving unit provides the mechanical power by changing the pressure within the pressure cavity.

In some implementations, the driving unit includes a pressure control mechanism, and the pressure control mechanism includes a pressure generator for changing an internal pressure of the liquid transfer cavity. Preferably, the pressure control mechanism further includes an air pressure detector for monitoring the internal pressure of the liquid transfer cavity.

In some implementations, the peritoneal dialysis system further includes an optional liquid supplementation tube and an optional last tube which are communicated with the liquid supply tube.

In some implementations, the peritoneal dialysis system further includes a waste fluid tube, wherein one end of the waste fluid tube is connected to the pressure cavity or the human body tube, and the other end thereof is connected to the waste fluid collector.

In some implementations, the peritoneal dialysis system further includes a pipeline switching valve assembly for controlling connection states of the human body tube and the medical solution bag pipeline.

In some implementations, the peritoneal dialysis system further includes a first weight detector for measuring a weight of a heating bag. Optionally, the peritoneal dialysis apparatus further includes a heating tray for supporting and heating the heating bag.

In some implementations, the peritoneal dialysis system further includes a second weight detector for measuring a weight of liquid in the pressure cavity.

Unless clearly contradictory, descriptions of any implementation of any aspect of the present invention can be combined in any manner with any other implementation of the same aspect or any implementation of another aspect to form a new implementation, and the combined implementation falls within the scope of the present invention.

### Beneficial Effects

In the peritoneal dialysis apparatus of the present invention, the liquid transfer cavity is in fluid communication with the driving unit through the connection interface, so that the driving unit is utilized to change the pressure within the liquid transfer cavity, thereby controlling the flow of liquid within the drainage pipeline. In the stages of gravity-based liquid supply and liquid supplementation, the driving unit is in an idle state, and only when mechanical power assistance is needed for medical solution infusion or drainage of waste fluid from the body is it necessary to change the pressure within the liquid transfer cavity through the driving unit, thereby controlling the liquid in the liquid pipeline to flow in a predetermined direction. Therefore, the driving unit participates in liquid flow driving only when needed, without continuous operation throughout the entire process. This saves energy and also reduces apparatus noise. In the drainage mode or powered infusion mode, by monitoring and controlling the pressure within the liquid transfer cavity, a stable flow rate is ensured, pressure surges are avoided, the treatment efficiency is high, and the patient experience is improved. In addition, when draining the body waste fluid, compared to existing gravity-based APD apparatuses, the use of negative pressure drainage is beneficial for complete waste fluid removal, ensuring the treatment efficacy. When infusing the medical solution, the gravity or powered infusion mode can be selected according to actual needs, so that the adaptability is strong and the stability is high.

Compared with powered APD designs employing a traditional membrane liquid cavity and labyrinth liquid channel design, the peritoneal dialysis apparatus of the present invention greatly simplifies a gas path control mechanism, has a simpler control principle, achieves faster and more stable drainage speed, and enables faster and more accurate liquid measurement; compared with the traditional gravity-based APD, the peritoneal dialysis apparatus of the present invention has high adaptability, achieves faster and more stable drainage speed, and yields a better treatment effect.

In summary, the peritoneal dialysis apparatus of the present invention has a simple control principle, a reliable structure, a faster and more stable drainage speed, a good treatment effect, is energy-saving and environmentally friendly, produces a low noise level, and can improve the treatment experience of the patient.

### BRIEF DESCRIPTION OF THE DRAWINGS

FIG. 1 is a schematic structural principle diagram of a peritoneal dialysis apparatus according to Embodiment 1.
FIG. 2 is a partial schematic structural diagram of the peritoneal dialysis apparatus in Embodiment 1.
FIG. 3 is a schematic diagram of a mounting height of the peritoneal dialysis apparatus according to Embodiment 1.
FIG. 4 is a schematic structural diagram of the peritoneal dialysis apparatus according to Embodiment 2.
FIG. 5 is a partial schematic structural diagram of the peritoneal dialysis apparatus in Embodiment 2.
FIG. 6 is a schematic structural diagram of the peritoneal dialysis apparatus according to Embodiment 3.
FIG. 7 is a partial schematic structural diagram of the peritoneal dialysis apparatus in Embodiment 3.

Reference numerals in the figures:
1-housing; 2-driving unit;
3-liquid transfer cavity, 31-medical solution bag pipeline, 32-drainage pipeline, 301-pressure cavity, 302-connection cavity, 303-connection interface, 304-liquid supply tube, 305-first branch tube, 306-second branch tube, 308-liquid supplementation tube, 309-last tube, 310-human body tube, 311-waste fluid tube;
4-heating bag, 5-first weight detector, 6-second weight detector;
7-pipeline switching valve assembly, 704-liquid supply tube switching valve, 705-first branch tube switching valve, 706-second branch tube switching valve, 708-liquid supplementation tube switching valve, 709-last tube switching valve, 710-human body tube switching valve, 711-waste fluid tube switching valve;
8-liquid supplementation bag, 9-last bag, 10-human peritoneal cavity, 11-waste fluid collector; 12-data processing unit;
h1-height of liquid supplementation bag or last bag, h2-height of human peritoneal cavity, h3-height of liquid transfer cavity, h4-height of heating bag, h5-height of waste fluid collector.

### DESCRIPTION OF THE EMBODIMENTS

To make the objectives, technical solutions, and advantages of the present invention clearer, the present invention will be further described below in conjunction with the accompanying drawings and embodiments. The same reference numbers in different drawings may indicate the same or similar structures.

As known to those skilled in the art, the peritoneal dialysis apparatus, also known as an automated peritoneal dialysis apparatus, APD, peritoneal dialysis equipment, automated peritoneal dialysis equipment, an automatic peritoneal dialysis machine, a peritoneal dialysis machine, an automated peritoneal dialysis machine, a peritoneal dialysis cycle machine or a machine for peritoneal dialysis, is a machine or apparatus for peritoneal dialysis treatment.

It is known to those skilled in the art that peritoneal dialysis medical solution, peritoneal dialysate, a peritoneal dialysis solution, a dialysate, or a medical solution refers to a medical solution used for peritoneal dialysis treatment. The medical solution that is to be drained out or has already been drained out after completion of the dwell within the human peritoneal cavity is referred to as a body waste fluid or waste fluid.

In the context of the present invention, a peritoneal dialysis medical solution bag, a PD medical solution bag, or a medical solution bag refers to a packaging bag containing a peritoneal dialysis medical solution, and unless otherwise specified, refers to the medical solution bag containing the medical solution inside. The medical solution bag may include a heating bag, a liquid supplementation bag, and a last bag. The heating bag refers to a medical solution bag placed on the heating tray of the peritoneal dialysis apparatus, capable of being heated and maintained at a desired temperature by the peritoneal dialysis apparatus. The liquid supplementation bag is a medical solution bag used to supplement a peritoneal dialysis medical solution to the heating bag.

The medical solution in the liquid supplementation bag usually has the same composition as the medical solution in the heating bag. The last bag is a medical solution bag containing the final infusion solution prescribed in a peritoneal dialysis treatment prescription, and the medical solution in the last bag is usually retained in the patient's abdominal cavity for a period of time after the treatment ends. The medical solution in the last bag may have the same or different composition as the medical solution in the heating bag or the liquid supplementation bag. One of ordinary skill in the art should understand that, depending on the physician's treatment prescription, the liquid supplementation bag is optional, and the last bag is also optional. In other words, there may or may not be a liquid supplementation bag, and there may or may not be a last bag.

In peritoneal dialysis treatment, clean peritoneal dialysis medical solution is required for each treatment. For medical safety reasons, a usually disposable liquid transfer cavity, including a cavity body portion and a pipeline portion connected to a cavity body, is required for each peritoneal dialysis treatment. Therefore, the liquid transfer cavity is a replaceable component, sometimes also referred to as a consumable. In some cases, the peritoneal dialysis apparatus includes the consumable portion; and in other cases, the peritoneal dialysis apparatus may not include the consumable portion, but must be combined with a matching consumable portion to function properly for peritoneal dialysis treatment.

### Embodiment 1: Peritoneal Dialysis Apparatus

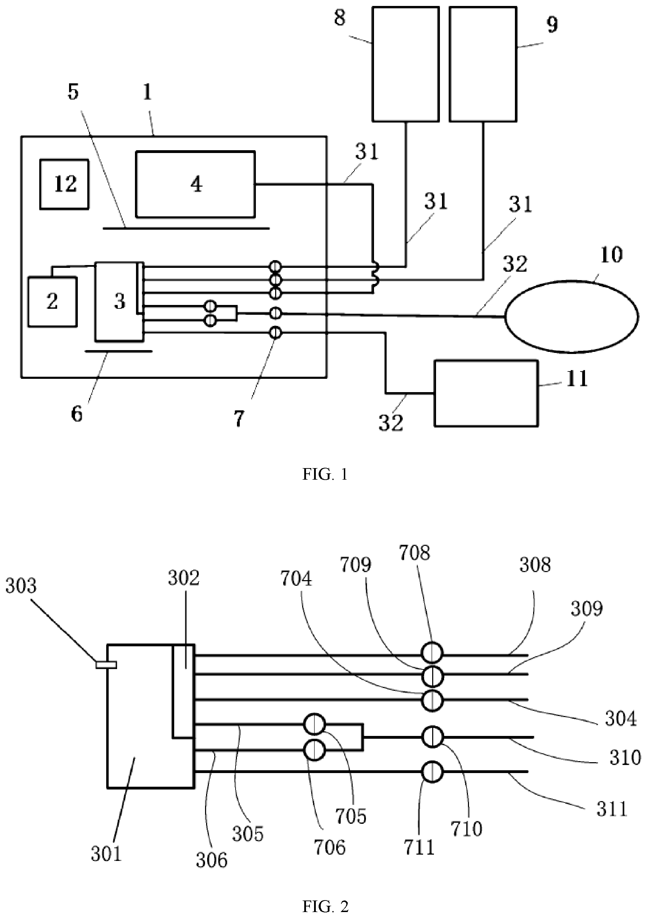

Referring to FIG. 1, this embodiment discloses a peritoneal dialysis apparatus, including a driving unit 2 and a replaceable liquid transfer cavity 3,
where the liquid transfer cavity 3 is connected to a liquid pipeline, and the liquid pipeline includes a medical solution bag pipeline 31 and a drainage pipeline 32;
and by changing the pressure in the liquid transfer cavity, the driving unit 2 controls the flow of liquid to enter and exit the liquid transfer cavity 3 through the liquid pipeline.

As shown in FIG. 1, the liquid transfer cavity 3 is connected to a heating bag 4, a waste fluid collector 11, and a human peritoneal cavity through the liquid pipelines respectively. Optionally, the waste fluid collector may be a waste fluid barrel or a waste fluid bag.

As shown in FIG. 2, the liquid transfer cavity 3 is provided with a connection interface 303, and the liquid transfer cavity 3 is in fluid communication with the driving unit 2 through the connection interface 303. The driving unit 2 can drive the flow of liquid within the drainage pipeline 32. Optionally, the driving unit 2 can also drive the flow of liquid within the medical solution bag pipeline 31.

In the peritoneal dialysis apparatus of the present invention, the liquid transfer cavity 3 is in fluid communication with the driving unit 2 through the connection interface 303, so that the driving unit 2 is utilized to change the pressure within the liquid transfer cavity, thereby controlling the flow of liquid within the liquid pipeline. Specifically, the connection interface 303 is a fluid communication interface, and gas can enter or exit the liquid transfer cavity through the connection interface.

In some implementations, referring to FIGS. 1 and 2, the liquid transfer cavity 3 is provided with a pressure cavity 301, and the connection interface 303 is disposed at a side wall of the pressure cavity 301. Therefore, the driving unit 2 is in fluid communication with the pressure cavity 301 through the connection interface 303. The driving unit 2 may include a gas control pipeline, and the connection interface 303 can be hermetically connected to the gas control pipeline.

In some implementations, gas can enter or exit the liquid transfer cavity 3 through the connection interface 303, and the driving unit 2 changes the pressure in the pressure cavity 301 by injecting gas into or withdrawing gas from the pressure cavity 301. By directly changing the pressure of a liquid cavity, rather than indirectly changing the pressure within the membrane liquid cavity through a control chamber in contact with a flexible membrane, the present invention greatly simplifies a gas path control principle and structure of the peritoneal dialysis apparatus, resulting in more stable and efficient operation.

Optionally, the connection interface may be provided with a gas-permeable and water-blocking membrane to prevent liquid from passing through the connection interface. Meanwhile, the bacteria-blocking membrane may be provided to prevent bacteria from entering the pressure cavity or the liquid pipeline with the gas through the connection interface. Specifically, the gas-permeable and water-blocking membrane and the bacteria-blocking membrane are the same layer of membrane, that is, the water-blocking membrane also serves as the bacteria-blocking membrane. In the context of the present invention, where applicable, for the sake of convenience and brevity of description, as shown in FIG. 2, the liquid transfer cavity 3 may specifically refer to the pressure cavity 301 of the liquid transfer cavity 3 without considering a pipeline portion connected to the pressure cavity 301. For example, the pressure in the liquid transfer cavity 3 may refer to the pressure in the pressure cavity 301, the weight of liquid in the liquid transfer cavity 3 may refer to the weight of liquid in the pressure cavity 301, and the waste fluid in the liquid transfer cavity 3 may refer to the waste fluid in the pressure cavity 301. Those skilled in the art will appreciate that such expressions are merely for convenience and brevity of description and will not cause any confusion or ambiguity.

In some embodiments, the driving unit 2 includes a pressure control mechanism, the pressure control mechanism includes a pressure generator and an air pressure detector, the pressure generator is configured to change the internal pressure of the pressure cavity 301, and the air pressure detector is configured to monitor the internal pressure of the pressure cavity 301. Specifically, the pressure control mechanism is hermetically connected to the gas control pipeline, and the pressure control mechanism is in fluid communication with the connection interface through the gas control pipeline.

Referring to FIGS. 1 and 2, the drainage pipeline 32 includes a human body tube 310 and a waste fluid tube 311. The liquid transfer cavity 3 may be connected to the human peritoneal cavity 10 through the human body tube 310, and communicated with the waste fluid collector 11 through the waste fluid tube 311.

As shown in FIG. 2, the liquid transfer cavity 3 may further include a connection cavity 302, and the connection cavity 302 is in fluid communication with one or more of the heating bag 4, the liquid supplementation bag 8, and the last bag 9 through the medical solution bag pipeline 31 respectively. The connection cavity 302 is not directly communicated with the pressure cavity 301, and the medical solution bag pipeline 31 is communicated with the human body tube 310 through the connection cavity 302.

The medical solution bag pipeline 31 includes a liquid supply tube 304, a liquid supplementation tube 308, and a last tube 309, wherein the connection cavity 302 is connected to the heating bag 4 through the liquid supply tube 304, connected to the liquid supplementation bag 8 through the liquid supplementation tube 308, and connected to the last bag 9 through the last tube 309. The liquid supplementation bag 8 and the last bag 9 are respectively connected to the heating bag 4 via the connection cavities 302 of the liquid transfer cavity 3.

Referring to FIGS. 1 and 2, the liquid transfer cavity 3 may also be in communication with the liquid supplementation bag 8 and the last bag 9 through the liquid supplementation tube 308 and the last tube 309, respectively.

As a first optional specific embodiment, referring to FIGS. 1 and 2, the drainage pipeline 32 includes a human body tube 310 and a waste fluid tube 311, wherein one end, close to the liquid transfer cavity, of the human body tube 310 is connected to the connection cavity 302 through a first branch tube 305, and connected to the pressure cavity 301 through a second branch tube 306, and the other end, away from the liquid transfer cavity 3, of the human body tube 310 is used to be connected to the human peritoneal cavity 10, one end of the waste fluid tube 311 is connected to the pressure cavity 301, and the other end of the waste fluid tube 311 is used to be connected to the waste fluid collector 11. Accordingly, the connection cavity 302 is in fluid communication with the human peritoneal cavity through the first branch tube 305 and the human body tube 310, the connection cavity 302 is in communication with the pressure cavity 301 through the first branch tube 305 and the second branch tube 306, and the pressure cavity 301 is connected to the waste fluid collector 11 through the waste fluid tube 311.

As shown in FIGS. 1 and 2, the peritoneal dialysis apparatus further includes a pipeline switching valve assembly 7, and the pipeline switching valve assembly 7 is configured to control communication or closure of the liquid pipeline.

Specifically, the pipeline switching valve assembly 7 may include a first branch tube switching valve 705, a second branch tube switching valve 706, a liquid supplementation tube switching valve 708, a last tube switching valve 709, a liquid supply tube switching valve 704, a human body tube switching valve 710, and a waste fluid tube switching valve 711. Ther pipeline switching valve assembly 7 can control corresponding pipelines to be communicated or not communicated. For example, the first branch tube switching valve 705 can control the communication or closure of the first branch tube 305.

As an alternative specific embodiment, the pipeline switching valve assembly 7 may include a solenoid valve or a motor-driven valve.

As shown in FIG. 1, the peritoneal dialysis apparatus may further include a first weight detector 5 for measuring a weight of the heating bag, and a heating tray for supporting and heating the heating bag 4, wherein the heating bag 4 is heated and kept warm by the heating tray so that a medical solution in the heating bag 4 reaches and is maintained at a desired temperature.

As shown in FIG. 1, the peritoneal dialysis apparatus may further include a second weight detector 6 for measuring an amount of liquid in the liquid transfer cavity 3. Without limitation, the peritoneal dialysis apparatus may also employ other existing techniques to measure the amount of liquid within the liquid transfer cavity, for example, using an optical measurement method.

Preferably, as shown in FIG. 1, the peritoneal dialysis apparatus further includes a housing 1, wherein both the first weight detector 5 and the second weight detector 6 are disposed in the housing 1. Optionally, a compartment for mounting the liquid transfer cavity may also be disposed within the housing 1. By arranging the pressure cavity and the weight detector within the housing, a measurement process can be protected from external interference, thereby improving the stability and reliability of liquid measurement.

In some embodiments, the peritoneal dialysis apparatus further includes a data processing unit 12, and the data processing unit 12 is configured to receive and process data from the first weight detector 5 and/or the second weight detector 6.

In some embodiments, the data processing unit 12 may be in communication link with the driving unit or the pressure control mechanism. Specifically, the data processing unit may receive pressure data from the pressure control mechanism or send pressure control signals to the pressure control mechanism.

In some implementations, the volume of the pressure cavity is independent of the pressure in the pressure cavity. Specifically, a cavity body portion of the liquid transfer cavity may be made of a rigid material, and a side wall of the pressure cavity does not undergo significant deformation within the working pressure range. This is significantly different from a flexible membrane side wall of a disposable cassette in the prior art.

In some implementations, the volume of the pressure cavity is 20-500 mL, preferably 50-200 mL, for example, about 100 mL.

### Peritoneal Dialysis Treatment

Peritoneal dialysis treatment generally includes multiple treatment cycles, each of which may include three stages: infusion, dwell, and drainage. Before the formal treatment cycles begin, an initial drainage is usually required. In some cases, a final infusion may also be performed after all treatment cycles are completed. Therefore, how to achieve infusion and drainage is key to enabling a peritoneal dialysis apparatus to perform peritoneal dialysis treatment.

In the medical solution infusion stage, the medical solution in the heating bag is infused into the human peritoneal cavity 10 through the liquid pipeline.

In the drainage stage, the waste fluid in the human peritoneal cavity is drained into the liquid transfer cavity 3, and then discharged to the waste fluid collector 11.

When the liquid supplementation bag or the last bag is required, the liquid in the liquid supplementation bag 8 or the last bag 9 usually needs to be transferred to the heating bag 4 and heated to a desired temperature.

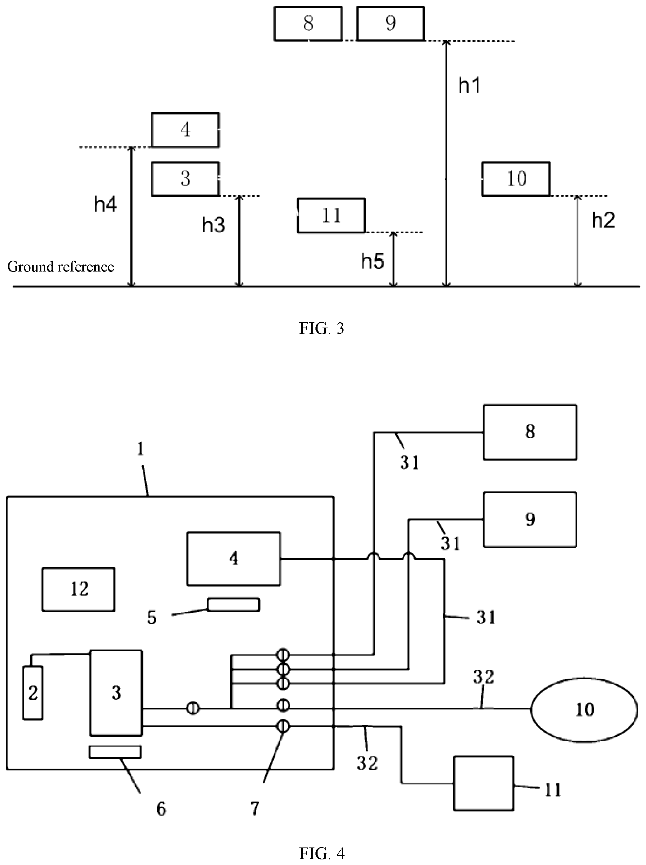

As shown in FIG. 3, a height h1 of the liquid supplementation bag 8 or the last bag 9 refers to a relative height from a bottom of the liquid supplementation bag 8 or the last bag 9 to the reference ground; a height h2 of the human peritoneal cavity 10 refers to a relative height from a bottom of the human peritoneal cavity 10 to the reference ground; a height h3 of the liquid transfer cavity 3 refers to a relative height from a bottom of the liquid transfer cavity 3 to the reference ground; a height h4 of the heating bag 4 refers to a relative height from a bottom of the heating bag 4 to the reference ground; and a height h5 of the waste fluid collector refers to a relative height from a bottom of the waste fluid collector to the reference ground. h1, h3, h4, and h5 are determined by a mounting position of the apparatus, a placement position of the medical solution bag, and a position of the waste fluid collector, and can be appropriately adjusted, and h2 is determined by a position and posture of a human body. Generally, h1 is significantly greater than h4, the relative positions of h4 and h3 are fixed with h4 being slightly greater than h3, and h5 is the smallest, but other configurations may also be adopted.

In one implementation, the height h2 of the human peritoneal cavity is less than the height h3 of the liquid transfer cavity and also less than the height h4 of the heating bag. In this case, a peritoneal dialysis machine of the present invention provides the best user experience.

In another implementation, the height h2 of the human peritoneal cavity is equal to or slightly greater than the height h3 of the liquid transfer cavity, but the height h2 of the human peritoneal cavity is less than the height h4 of the heating bag.

The peritoneal dialysis apparatus of the present invention primarily uses a gravity-based infusion mode to achieve medical solution infusion. Specifically, as shown in FIGS. 1 and 2, the liquid supply tube switching valve 704, the first branch tube switching valve 705, and the human body tube switching valve 710 are opened while other pipeline switching valve assemblies are kept in a closed state, and the liquid supply tube 304, the first branch tube 305, and the human body tube 310 are opened, and since the height h2 of the human peritoneal cavity is less than the height h4 of the heating bag, the medical solution in the heating bag 4 is infused by gravity into the human peritoneal cavity 10 connected to the human body tube 310.

The peritoneal dialysis apparatus of the present invention can also use powered infusion to achieve medical solution infusion, for example, in a case where the height h2 of the human peritoneal cavity is greater than or equal to the height h4 of the heating bag, or in a case where gravity-based infusion is not smooth or an infusion speed cannot meet the requirement although h2 is less than h4.

In the powered infusion mode, firstly, the liquid supply tube switching valve 704, the first branch tube switching valve 705, and the second branch tube switching valve 706 are opened while other pipeline switching valve assemblies are kept in a closed state, the liquid supply tube 304, the first branch tube 305, and the second branch tube 306 are opened, the pressure in the pressure cavity 301 is adjusted through the driving unit 2 to create negative pressure in the pressure cavity 301, a medical solution in the heating bag 4 flows into the pressure cavity 301 under the action of the negative pressure, then the liquid supply tube switching valve 704 and the first branch tube switching valve 705 are closed, the pressure in the pressure cavity 301 is adjusted to restore it to atmospheric pressure, and then the human body tube switching valve 710 is opened to open the human body tube 310, the pressure in the pressure cavity 301 is adjusted to a positive pressure, and the medical solution in the pressure cavity 301 flows into the human peritoneal cavity under the action of the pressure.

The peritoneal dialysis apparatus of the present invention can adopt a powered drainage mode to perform the drainage process. Specifically, while keeping other pipeline switching valve assemblies in a closed state, firstly, the second branch tube switching valve 706 and the human body tube switching valve 710 are opened, to communicate the human peritoneal cavity 10 with the pressure cavity 301; then, the pressure in the pressure cavity 301 is adjusted to create negative pressure in the pressure cavity 301, and a waste fluid in the human peritoneal cavity is drained into the pressure cavity 301 under the action of pressure; when the waste fluid in the pressure cavity 301 reaches a set amount or the waste fluid in the human peritoneal cavity is emptied, all pipeline switching valve assemblies are closed and the pressure in the pressure cavity 301 is adjusted to atmospheric or positive pressure; then, the waste fluid tube switching valve 711 and the waste fluid tube 311 are opened, and the waste fluid in the pressure cavity 301 is discharged into the waste fluid collector 11 by gravity or under pressure.

In some cases, peritoneal dialysis treatment requires the use of the liquid supplementation bag and/or the last bag. Liquid in the liquid supplementation bag or the last bag needs to be transferred to the heating bag and heated to a desired temperature before it can be infused into the human body. The peritoneal dialysis apparatus of the present invention mainly uses a gravity-based liquid supplementation mode for liquid supplementation. Taking gravity-based liquid supplementation from the liquid supplementation bag as an example, as shown in FIGS. 1, 2, and 3, the liquid supply tube switching valve 704 and the liquid supplementation tube switching valve 708 are opened, while other pipeline switching valve assemblies are kept in a closed state, and the liquid supply tube 304 and the liquid supplementation tube 308 are opened. Since the height h1 of the liquid supplementation tube is greater than the height h4 of the heating bag, the medical solution in the liquid supplementation bag 8 flows into the heating bag 4 by gravity. The gravity-based liquid supplementation mode of the last bag is basically the same as the gravity-based liquid supplementation mode of the liquid supplementation bag and will not be repeated.

In some implementations, when the gravity-based liquid supplementation mode fails to meet the liquid supplementation requirement, a powered liquid supplementation mode can also be adopted. For example, by controlling the pipeline switching valve assembly 7 to communicate the liquid supplementation bag 8 with the pressure cavity 301, the medical solution in the liquid supplementation bag 8 can be transferred into the pressure cavity 301 by gravity or by adjusting the pressure in the pressure cavity 301; then, by controlling the pipeline switching valve assembly 7 to communicate the heating bag 4 with the pressure cavity 301, the medical solution in the pressure cavity 301 can be transferred into the heating bag 4 by adjusting the pressure in the pressure cavity 301. The powered liquid supplementation mode of the last bag is basically the same as the powered liquid supplementation mode of the liquid supplementation bag and will not be repeated.

In the peritoneal dialysis apparatus of the present invention, in the stages of gravity-based liquid supply and liquid supplementation, no mechanical power is required and the driving unit 2 is in an idle state; and only when power assistance is needed for medical solution infusion or drainage of waste fluid from the body is it necessary to change the pressure within the liquid transfer cavity through the driving unit 2, thereby controlling the liquid in the liquid pipeline to flow in a predetermined direction. Therefore, the driving unit 2 participates in liquid flow driving only when needed, without continuous operation throughout the entire process. This saves energy and also reduces apparatus noise. In addition, when draining the body waste fluid, compared to existing gravity-based APD apparatuses, the use of negative pressure drainage is beneficial for complete waste fluid removal, ensuring the treatment efficacy. When infusing the medical solution, infusion may also be automatically performed by gravity or powered mode depending on conditions. In the drainage mode or powered infusion mode, by monitoring and controlling the pressure within the liquid transfer cavity, a stable flow rate can be ensured, pressure surges are avoided, the treatment efficiency is high, and the patient experience is improved.

Compared with powered APD designs employing a traditional membrane liquid cavity and labyrinth liquid channel design, the peritoneal dialysis apparatus of the present invention greatly simplifies a gas path control mechanism, has a simpler control principle, and enables more accurate liquid measurement; compared with the traditional gravity-based APD, the peritoneal dialysis apparatus of the present invention achieves faster and more stable drainage speed, and yields a better treatment effect.

### Embodiment 2: Peritoneal Dialysis Apparatus

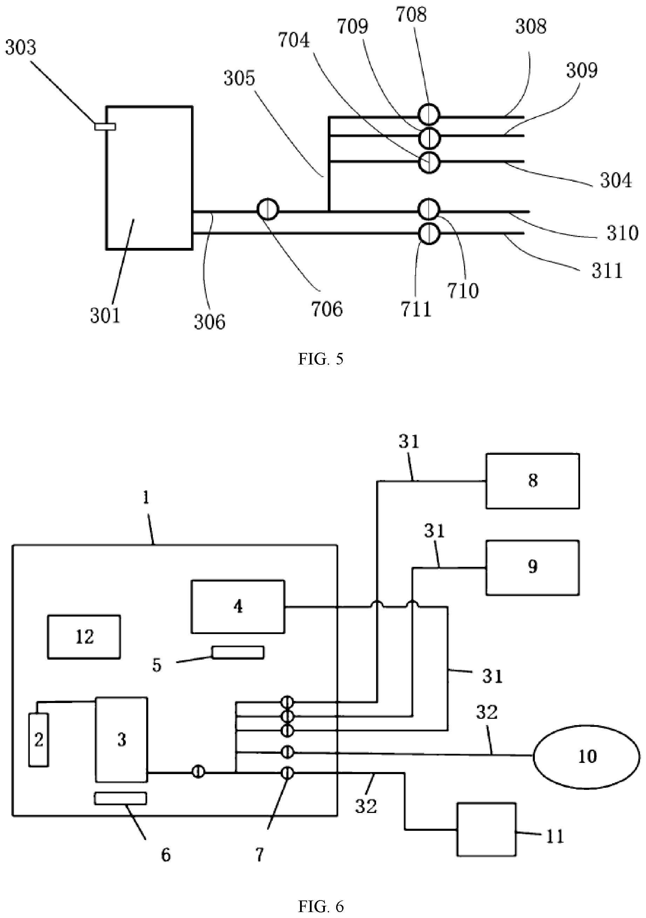

Referring to FIGS. 4 and 5, Embodiment 2 differs from Embodiment 1 in that a structure of the liquid transfer cavity 2 in Embodiment 3 is different from a structure of the liquid transfer cavity in Embodiment 1. Accordingly, the number and positions of the pipeline switching valve assemblies 7 in Embodiment 2 are also different from those in Embodiment 1.

Specifically, the liquid transfer cavity 3 in Embodiment 2 is provided with a pressure cavity 301, and the pressure cavity 301 is in fluid communication with the driving unit 2 through the connection interface 303.

As shown in FIGS. 4 and 5, the drainage pipeline 32 includes a human body tube 310 and a waste fluid tube 311, wherein the human body tube 310 is configured to be connected to the human peritoneal cavity 10, the human body tube 310 is connected to the medical solution bag pipeline 31 and the pressure cavity 301 through branch tubes, and the pressure cavity 301 is connected to the waste fluid collector 11 through the waste fluid tube 311.

In this embodiment, the waste fluid tube 311 is directly connected to the pressure cavity 301.

The liquid pipeline in Embodiment 2 includes branch tubes, and the branch tubes include a first branch tube 305 and a second branch tube 306. One end of the second branch tube 306 is connected to the pressure cavity 301, and one end, away from the pressure cavity 301, of the second branch tube 306 is connected to the first branch tube 305, and the first branch tube 305 is provided with a first branch interface, a second branch interface, a third branch interface, and a fourth branch interface, wherein the first branch interface is connected to the heating bag 4 through the liquid supply tube 304, the second branch interface is connected to the liquid supplementation bag 8 through the liquid supplementation tube 308, the third branch interface is connected to the last bag 9 through the last tube 309, and the fourth branch interface is connected to the human peritoneal cavity 10 through the human body tube 310. In particular, the first branch tube 305, the second branch tube 306, and the human body tube 310 are communicated with each other at the fourth branch interface.

The pipeline switching valve assembly 7 can control the opening or closing of the second branch tube 306, the liquid supplementation tube 308, the last tube 309, the liquid supply tube 304, the waste fluid tube 311, and the human body tube 310. For example, the pipeline switching valve assembly 7 may include a second branch tube switching valve 706, a liquid supplementation tube switching valve 708, a last tube switching valve 709, a liquid supply tube switching valve 704, a human body tube switching valve 710, and a waste fluid tube switching valve 711.

The implementation method of peritoneal dialysis treatment in the present invention is similar to that in Embodiment 1, wherein the opening or closing of pipelines is achieved by controlling the pipeline switching valve assembly 7, thereby achieving gravity-based infusion or gravity-based liquid supplementation. By controlling the pipeline switching valve assembly 7 to open or close the pipelines, and by adjusting the pressure within the pressure cavity 301 by the driving unit 2, powered infusion, powered drainage, or powered liquid supplementation can be achieved.

The liquid transfer cavity of the present invention is a single-cavity structure without a connection cavity, having a simpler structure and being easier to manufacture. Meanwhile, the number of pipeline switching valve assemblies 7 in the present invention can be reduced.

### Embodiment 3: Peritoneal Dialysis Apparatus

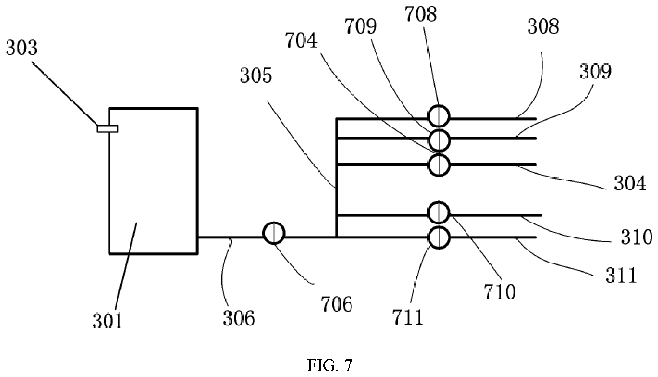

Referring to FIGS. 6 and 7, Embodiment 3 differs from Embodiment 1 in that a structure of the liquid transfer cavity 3 in Embodiment 3 is different from a structure of the liquid transfer cavity in Embodiment 1. Accordingly, the number and positions of the pipeline switching valve assemblies 7 in Embodiment 3 are also different from those in Embodiment 1.

The liquid transfer cavity 3 in Embodiment 3 is provided with a pressure cavity 301, and the pressure cavity 301 is in fluid communication with the driving unit 2 through the connection interface 303.

As shown in FIGS. 6 and 7, the drainage pipeline 32 includes a human body tube 310 and a waste fluid tube 311, wherein the human body tube 310 is configured to be connected to the human peritoneal cavity 10, the human body tube 310 is connected to the medical solution bag pipeline 31 and the pressure cavity 301 through branch tubes, and the pressure cavity 301 is connected to the waste fluid collector 11 through the waste fluid tube 311.

In this embodiment, the waste fluid tube 311 is connected to the pressure cavity 301 through the branch tube.

The liquid pipeline in Embodiment 3 includes branch tubes, the branch tubes include a first branch tube 305 and a second branch tube 306. One end of the second branch tube 306 is connected to the pressure cavity 301, one end, away from the pressure cavity 301, of the second branch tube 306 is connected to the first branch tube 305, and the first branch tube 305 is provided with a first branch interface, a second branch interface, a third branch interface, a fourth branch interface, and a fifth branch interface, wherein the first branch interface is connected to the heating bag 4 through the liquid supply tube 304, the second branch interface is connected to the liquid supplementation bag 8 through the liquid supplementation tube 308, the third branch interface is connected to the last bag 9 through the last tube 309, the fourth branch interface is connected to the human peritoneal cavity 10 through the human body tube 310, and the fifth branch interface is connected to the waste fluid collector 11 through the waste fluid tube 311. In particular, the first branch tube 305, the second branch tube 306, and the waste fluid tube 311 are communicated with each other at the fifth branch interface. Alternatively, the first branch tube 305 and the second branch tube 306 may be connected at the fourth branch interface, other branch interfaces, or other positions.

The pipeline switching valve assembly 7 can control the opening or closing of the second branch tube 306, the liquid supplementation tube 308, the last tube 309, the liquid supply tube 304, the waste fluid tube 311, and the human body tube 310. For example, the pipeline switching valve assembly 7 may include a second branch tube switching valve 706, a liquid supplementation tube switching valve 708, a last tube switching valve 709, a liquid supply tube switching valve 704, a human body tube switching valve 710, and a waste fluid tube switching valve 711.

The implementation method of peritoneal dialysis treatment in the present invention is similar to that in Embodiment 1, wherein the opening or closing of pipelines is achieved by controlling the pipeline switching valve assembly 7, thereby achieving gravity-based infusion or gravity-based liquid supplementation. By controlling the pipeline switching valve assembly 7 to open or close the pipelines, and by adjusting the pressure within the pressure cavity 301 by the driving unit 2, powered infusion, powered drainage, or powered liquid supplementation can be achieved.

The liquid transfer cavity of the present invention is a single-cavity structure without a connection cavity, having a simpler structure and being easier to manufacture. Meanwhile, the number of pipeline switching valve assemblies 7 required in the present invention can be reduced.

### Embodiment 4: Liquid Measurement Method

Referring to FIG. 1, FIG. 4, or FIG. 6, in the present invention, the first weight detector 5 is used to measure the weight of the heating bag 4, and the second weight detector 6 is used to measure the weight of the liquid in the liquid transfer cavity 3, and data from the first weight detector 5 and the second weight detector 6 are used for liquid measurement.

This embodiment discloses a liquid measurement method for a peritoneal dialysis apparatus, including:
S21: draining a waste fluid to be drained in a human peritoneal cavity into a liquid transfer cavity in batches;
S22: collecting a weight value of each batch of waste fluid respectively, and after the weight value of each batch of waste fluid is collected, discharging the batch of waste fluid in the liquid transfer cavity into a waste fluid collector; and
S23: calculating a drainage amount based on the weight values of the batches of waste fluid.

In this embodiment, the waste fluid to be drained from the human peritoneal cavity is drained in batches into the pressure cavity of the liquid transfer cavity; subsequently, the weight value of each batch of waste fluid is collected in separate times, so as to cumulatively obtain the amount of waste fluid discharged from the human peritoneal cavity. By measuring the waste fluid in separate times, a measuring range of the second weight detector can be reduced, and the second weight detector can use a small-range weight detection element, thus achieving higher measurement accuracy and lower cost. Secondly, the way of measuring the waste fluid in separate times also allows the liquid transfer cavity to use a smaller capacity and volume, occupying less space. The second weight detector is disposed below the liquid transfer cavity, and the waste fluid collector can be flexibly arranged as needed without interfering with the measurement of the waste fluid, making installation and use of the apparatus more convenient and flexible.

In the step S22, the step of collecting a weight value of each batch of waste fluid respectively may include:
collecting the weight value of each batch of waste fluid by using the second weight detector.

In the step S23, the step of calculating a drainage amount based on the weight value of each batch of waste fluid includes:
cumulatively summing the weight values of the batches of waste fluid, thereby obtaining an amount of waste fluid discharged from the human peritoneal cavity.

Specifically, after the data processing unit 12 receives the weight values of the batches of waste fluid collected by the second weight detector 6, it cumulatively sum the weight values of the batches of waste fluid to obtain the amount of waste fluid discharged from the human peritoneal cavity 10.

The liquid measurement method may further include:
S11: collecting a weight value of a heating bag in real time to obtain a volume of a medical solution infused into the human peritoneal cavity in real time.

The amount of the medical solution infused into the human peritoneal cavity is also commonly referred to as an infusion amount. By means of the drainage amount and the infusion amount, an ultrafiltration amount of peritoneal dialysis treatment can also be calculated. For example, the ultrafiltration amount can be obtained by subtracting the infusion amount from the drainage amount.

In the step S11, the step of collecting a weight value of a heating bag may include:
collecting a weight value of a heating bag in real time by means of a first weight detector disposed below the heating bag, sending, by the first weight detector, the collected weight value of the heating bag to a data processing unit, and processing, by the data processing unit, the collected weight value of the heating bag to obtain an amount of liquid infused into the human peritoneal cavity.

For example, an original weight value of the heating bag before the medical solution is infused into the human body is W₀, and the currently collected weight value of the heating bag is W₁, then an amount of liquid infused into the human peritoneal cavity Wₜ=W₀-W₁.

Preferably, the first weight detector and the second weight detector may be disposed within the housing of the peritoneal dialysis apparatus, thereby reducing interference from external factors on the weighing measurement and improving the reliability of liquid measurement.

### Embodiment 5: Weighing Verification Method

Referring to FIG. 1, FIG. 4, or FIG. 6, in the present invention, the first weight detector 5 is used to measure the weight of the heating bag 4, and the second weight detector 6 is used to measure the weight of the liquid in the liquid transfer cavity 3, and data from the first weight detector 5 and the second weight detector 6 are used for weighing verification.

This embodiment discloses a weighing verification method for a peritoneal dialysis apparatus, the method including:
S201: delivering at least part of a medical solution in a heating bag to a liquid transfer cavity, so that weight values of the heating bag and the liquid transfer cavity change;
S202: collecting the weight value of the heating bag with a first weight detector;
S203: collecting the weight value of the liquid transfer cavity with a second weight detector; and
S204: verifying whether there is a weighing abnormality between the first weight detector and the second weight detector based on the weight value of the heating bag and the weight value of the liquid transfer cavity.

The step S202 and the step S203 may each include collecting a plurality of said weight values.

It should be noted that the number of execution times and the sequence of the steps are not particularly limited; for example, the step S201, the step S202, and the step S203 may be performed simultaneously, and then the step S204 is performed. Alternatively, for example, the step S202 and the step S203 may be performed first, then the step S201 is performed, thereafter the step S202 and the step S203 are performed again, and finally the step S204 is performed.

In this embodiment, mutual verification between two weighing sensors, namely, the first weight detector and the second weight detector, effectively eliminates measurement errors caused by a failure of the weighing sensor, thus greatly improving the system reliability and the user safety. Since the weighing verification process does not require the medical solution to be discharged into the waste fluid collector or the waste fluid tube, so that the medical solution is prevented from being wasted.

In addition, both the first weight detector and the second weight detector may be disposed within the housing of the peritoneal dialysis apparatus, thereby reducing interference from external factors on weighing and improving the reliability and speed of verification.

As an optional specific embodiment, the method further includes:
if a weighing abnormality exists between the first weight detector and the second weight detector, sending a warning message to a user. The warning message may be sent to the user through a warning device, and the warning message may be notified to the user by means of voice playback, buzzing, indicator light display, display screen display, vibration, etc. The warning device may be any one or a combination of a speaker, a buzzer, an indicator light, a vibration motor, or a display. In the description of the present invention, unless the context clearly conflicts or is obviously contrary to the spirit of the present invention, reference to the liquid transfer cavity may indicate the pressure cavity of the liquid transfer cavity without considering the pipeline portion or connection cavity connected to the pressure cavity, for example, "the pressure in the liquid transfer cavity" may refer to the pressure in the pressure cavity of the liquid transfer cavity; "the weight of liquid in the liquid transfer cavity" may refer to the weight of liquid in the pressure cavity of the liquid transfer cavity; "fluid communication with the liquid transfer cavity" may refer to fluid communication with the pressure cavity of the liquid transfer cavity; and "delivering or draining liquid to the liquid transfer cavity" may refer to delivering the medical solution to the pressure cavity of the liquid transfer cavity or draining the body waste fluid to the pressure cavity of the liquid transfer cavity. Those skilled in the art will appreciate that such expressions are merely for convenience and brevity of description and will not cause any confusion or ambiguity.

In the context of the present invention, a technical feature without a specific number may refer to one, two or more of the technical features. The conjunction "or" used to describe a technical feature has the same meaning as "and/or" unless it is theoretically impossible or clearly contradicted by context. For example, "A or B or C" may include "A", "B", "C", "A and B", "A and C", "B and C", and "A and B and C".

It should be noted that, provided there is no obvious contradiction, the apparatuses, systems, and methods involved in the foregoing embodiments or implementations may be implemented independently as a single embodiment or implementation, or may be implemented in combination of multiple (two or more) embodiments or implementations, and these implementations may be applied individually or in combination to existing automated peritoneal dialysis apparatuses; and the various optional specific implementations included in the embodiments may be combined arbitrarily without particular limitation.

The foregoing descriptions are merely preferred embodiments of the present invention and are not intended to limit the present invention in any way. Although the present invention has been disclosed above by way of preferred embodiments, it is not intended to limit the present invention. Any person skilled in the art may utilize the technical contents suggested above to make alterations or modifications to produce equivalent embodiments incorporating equivalent variations. Any simple modifications, equivalent variations, and modifications made to the above embodiments in accordance with the technical solution of the present invention, without departing from the content of the technical solutions of the present invention, shall still fall within the scope of the present invention.

## Claims

1. A peritoneal dialysis apparatus for being used in combination with a liquid transfer cavity for peritoneal dialysis, the peritoneal dialysis apparatus comprising a driving unit, wherein
the liquid transfer cavity comprises a pressure cavity, the pressure cavity is in fluid communication with the driving unit, and by changing a pressure in the pressure cavity, the driving unit controls liquid to enter and exit the pressure cavity through a liquid pipeline.

2. The peritoneal dialysis apparatus according to claim 1, wherein the driving unit changes the pressure in the pressure cavity by injecting gas into or withdrawing gas from the pressure cavity.

3. The peritoneal dialysis apparatus according to claim 1, wherein the liquid comprises either or both of a peritoneal dialysis medical solution and a body waste fluid.

4. The peritoneal dialysis apparatus according to claim 1, wherein the driving unit comprises a pressure control mechanism, the pressure control mechanism comprises a pressure generator and an air pressure detector, the pressure generator is configured to change the pressure in the pressure cavity, and the air pressure detector is configured to monitor the pressure in the pressure cavity.

5. The peritoneal dialysis apparatus according to claim 1, wherein the peritoneal dialysis apparatus further comprises a pipeline switching valve assembly, and the pipeline switching valve assembly is configured to control communication or closure of the liquid pipeline.

6. The peritoneal dialysis apparatus according to claim 1, wherein the driving unit operates at least in a drainage process to discharge the body waste fluid out of a human body.

7. The peritoneal dialysis apparatus according to claim 1, wherein the operation of the driving unit is not essential in a medical solution infusion process.

8. The peritoneal dialysis apparatus according to claim 1, wherein the peritoneal dialysis apparatus further comprises a first weight detector for measuring a weight of a heating bag.

9. The peritoneal dialysis apparatus according to claim 1, wherein the peritoneal dialysis apparatus further comprises a second weight detector for measuring a weight of the liquid in the pressure cavity.

10. The peritoneal dialysis apparatus according to claim 1, wherein the peritoneal dialysis apparatus further comprises a housing, and a compartment for mounting the liquid transfer cavity is disposed within the housing.

11. The peritoneal dialysis apparatus according to claim 1, wherein the liquid pipeline comprises a medical solution bag pipeline, a human body tube, and branch tubes, the medical solution bag pipeline and the human body tube are connected to the pressure cavity through a same branch tube, and the medical solution bag pipeline is capable of communicating with the human body tube without passing through the pressure cavity.

12. The peritoneal dialysis apparatus according to claim 11, wherein the liquid transfer cavity further comprises a connection cavity, the connection cavity is not directly communicated with the pressure cavity, and the medical solution bag pipeline is communicated with the human body tube through the connection cavity.

13. The peritoneal dialysis apparatus according to claim 11, wherein the liquid pipeline further comprises a waste fluid tube, and the waste fluid tube is directly connected to the pressure cavity.

14. The peritoneal dialysis apparatus according to claim 1, wherein a volume of the pressure cavity remains substantially unchanged within a working pressure range of the pressure cavity.

15. The peritoneal dialysis apparatus according to claim 1, wherein a gas-permeable and water-blocking element is disposed at a position where the pressure cavity is connected to the driving unit.
